# EUROPEAN PATENT APPLICATION

(11) **EP 3 721 890 A1**
(43) Date of publication of application: **14.10.2020**
(21) Application number: 18885969.8
(22) Date of filing: 08.05.2018
(51) Int. Cl.: A61K 36/258, A61P 27/02, A61P 27/04

(54) **OPHTHALMIC DRUG PREPARATION AND USES THEREOF**

(30) Priority: 04.12.2017 CN 201711262528
(71) Applicant: China Pharmaceutical University, District Nanjing Jiangsu 211198 (CN)
(72) Inventor: TAN, Ninghua, Nanjing Jiangsu 210096 (CN); WANG, Zhen, Nanjing Jiangsu 210096 (CN); TANG, Kai, Nanjing Jiangsu 210096 (CN)
(74) Representative: Hanna Moore + Curley
(86) International application number: PCT/CN2018/085962
(87) International publication number: WO 2019/109590

(57) **Abstract**

The present disclosure discloses an ophthalmic pharmaceutical preparation as well as a preparation method and application thereof. Components of the pharmaceutical preparation are easy to obtain and are from a panax notoginseng extract. The pharmaceutical preparation is a natural traditional Chinese medicine preparation and contains the following components with a total of 45 to 95%: a notoginsenoside R1, a notoginsenoside R2, a ginsenoside Rg1, a ginsenoside Rg2, a ginsenoside Re, a ginsenoside Rb1, a ginsenoside Rb2, a ginsenoside Rd, a ginsenoside Rf, a ginsenoside Rc, a ginsenoside Rh1 and a gypenoside IX. The extract can be made into eye drops, eye ointments, eye pads, eye cream and other various dosage forms according to clinical demands. Pharmacological experiments prove that the drug is safe and effective without stimulation, and has a curative effect on damages to eyes and vision caused by eye dryness, distending pain, asthenopia, eye angiosclerosis and the like, thereby having a better clinical application prospect.

## Description

### Technical Field

The present disclosure belongs to a pharmaceutical technology, and particularly relates to a pharmaceutical preparation for preventing and treating eye fatigue, eye dryness and distending pain and eye angiosclerosis as well as application thereof.

### Background Art

With the progress of society, the smooth communication of the Internet as well as the popularization and wide application of paperless office and screen products such as smartphones and televisions, the work, study and lifestyles of people have changed, and staring at a screen has become a basic habit. If staring at a flickering screen at short distance for a long time, eye ciliary muscle contracts for a long time without relaxing, which easily causes diseases such as eye dryness, distending pain, asthenopia and eye angiosclerosis, damaging the eyes and vision.

Panax notoginseng is sweet and slightly bitter in taste, and warm in nature, and has the effects of activating blood circulation to dissipate blood stasis, eliminating swelling, relieving pain, and stopping bleeding and healing. Modern science has proven that the panax notoginseng contains ginsenoside and notoginsenoside components, and thus can obviously shorten bleeding and blood coagulation time and have a good effect of stopping bleeding; can promote division growth and number increasing of various blood cells, promote proliferation and release processes of bone marrow cells, increase the number and activity of bone marrow erythrocytes, and thus achieve a function of promoting hematopoiesis; can significantly inhibit formation of aortic intimal plaques of experimental atherosclerosis rabbits; can excite nervous centralis, improve mental and physical power, and thus shows fatigue resistance; can significantly improve the phagocytic ratio and phagocytic index of macrophages of mice, increase the sum of leukocytes in peripheral blood, and reduce the movement index of the leukocytes; can obviously inhibit all of increasing of capillary permeability, inflammatory exudation, tissue edema, leukocyte migration as well as late granulation tissue hyperplasia caused by acute inflammations; and can reduce lipid peroxides in an organism, and thus has an anti-aging effect. Various pharmacological activities of the panax notoginseng are used for treatment of various cardiovascular and cerebrovascular diseases. An extract of the panax notoginseng is made into drugs such as "Xuesaitong", "Guanxinning" and "Xueshuantong" on the market. The effect of activating blood circulation to dissipate blood stasis of the panax notoginseng is also widely applied to traumatic injury drugs, so that strain muscle tissues are recovered. However, there has been no panax notoginseng product for eye ciliary muscle fatigue, asthenopia and other eye problems on the market.

Many Chinese patents for asthenopia are disclosed, many of which use the panax notoginseng for component preparation. For example, the patent CN103099977B discloses the invention of "Externally Used Ophthalmic Drug Composition and Use of Externally Used Ophthalmic Drug Composition in Application of Treatment of Uveitis", and the composition is prepared into an eye drop from a pimecrolimus eye drop, a panax notoginseng ginsenoside Rb1, a fructus forsythiae extract aqueous solution and a herba imperatae extract aqueous solution, which is used for ophthalmic treatment of uveitis. The patent CN103520548B discloses the invention of "Asthenopia Improving Pharmaceutical Composition and Preparation Method thereof', and the composition is prepared into an oral preparation prepared from fructus lycii, semen cassiae, radix morindae officinalis, radix glycyrrhizae, radix notoginseng, radix puerariae, herba lophatheri, fructus citri sarcodactylis and the like, which is used as a drug for relieving asthenopia. The patent application CN102960713A discloses the invention of "Health Care Product for Controlling Screen Contamination", and the composition is prepared into an oral preparation from phaseolus calcaratus, dictyophora phalloidea, pseudo-ginseng, American ginseng, pork liver, Chinese anisopappus flowers, cortex moutan, radix rehmanniae, fructus lycii, poria cocos, fructus crataegi, selenium-rich green tea, radix astragali, and fructus mori is used for eliminating contamination to a human body by various electronic products, and preventing and treating damages to eyes, hematopoiesis, and nervous and immune systems of the human body caused by electronic screens, etc. Currently, there are no pharmaceutical preparations for preventing and treating eye fatigue, eye dryness and distending pain and eye angiosclerosis by using notoginsenosides as well as new dosage forms thereof.

### Summary of the Invention

The present disclosure is directed to provide an ophthalmic pharmaceutical preparation for preventing and treating asthenopia, eye dryness and distending pain, and eye angiosclerosis as well as a preparation method and application thereof.

In order to realize the above objectives of the present disclosure, the present disclosure provides the following technical solutions.

Provided is an ophthalmic pharmaceutical preparation, an effective component of the ophthalmic pharmaceutical preparation is a notoginsenoside extract. The notoginsenoside extract is prepared and extracted from panax notoginseng.

The notoginsenoside extract contains the following components with a total content of 45 to 95%: a notoginsenoside R1, a notoginsenoside R2, a ginsenoside Rg1, a ginsenoside Rg2, a ginsenoside Re, a ginsenoside Rb1, a ginsenoside Rb2, a ginsenoside Rd, a ginsenoside Rf, a ginsenoside Rc, a ginsenoside Rh1 and a gypenoside IX.

Further preferably, the notoginsenoside extract contains the following components with a total content of 75 to 90%: the notoginsenoside R1, the ginsenoside Rg1, the ginsenoside Re, the ginsenoside Rb1 and the ginsenoside Rd.

Further, in the notoginsenoside extract, a content of the notoginsenoside R1 (C₄₇H₈₀O₁₈) is equal to or greater than 3.0%. A content of the ginsenoside Rg1 (C₄₂H₇₂O₁₄) is equal to or greater than 15.0%. A content of the ginsenoside Re (C₄₈H₈₂O₁₈) is equal to or greater than 1.0%. A content of the ginsenoside Rb1 (C₅₄H₉₂O₂₃) is equal to or greater than 20.0%. A content of the ginsenoside Rd (C₄₈H₈₂O₁₉) is equal to or greater than 2.5%.

Furthermore, in the notoginsenoside extract, the content of the notoginsenoside R1 (C₄₇H₈₀O₁₈) is equal to or greater than 5.0%. The content of the ginsenoside Rg1 (C₄₂H₇₂O₁₄) is equal to or greater than 25.0%. The content of the ginsenoside Re (C₄₈H₈₂O₁₈) is equal to or greater than 2.5%. The content of the ginsenoside Rb1 (C₅₄H₉₂O₂₃) is equal to or greater than 30.0%. The content of the ginsenoside Rd (C₄₈H₈₂O₁₉) is equal to or greater than 5%.

As a preferred solution, a weight percentage content of the notoginsenoside extract in the pharmaceutical preparation is 0.1 to 30%.

Further, the weight percentage content of the notoginsenoside extract in the pharmaceutical preparation is 0.2 to 10%.

Dosage forms of the preparation are collyria, eye drops, eye ointments or eye pads. The pads may be prepared by immersing or loading the prepared eye drops and eye ointments onto thin film materials.

As another preferred solution, the notoginsenoside extract in the pharmaceutical preparation is the only effective component, and the pharmaceutical preparation is prepared from 0.1 to 30% by weight of the notoginsenoside extract and 70 to 99.9% by weight of a pharmaceutically acceptable carrier and/or excipient. Further, the pharmaceutical preparation is prepared from 0.2 to 10% by weight of the notoginsenoside extract and 90 to 99.8% by weight of the pharmaceutically acceptable carrier and/or excipient.

The pharmaceutically acceptable carrier is selected from one or more of water, normal saline, vaseline, liquid paraffin, lanolin and glycerine.

As yet another preferred solution, for the pharmaceutical preparation, the effective component, the notoginsenoside extract, is added into artificial tears or various eye drop drugs with a therapeutical effect. The artificial tears are known to those of skill in the art, and may be composed of one or more of anhydroglucose-70, polyethylene glycol 400, a sodium carboxymethylcellulose eye drop, vitamin A, carbomer sorbic acid, sodium hyaluronate, polyvinyl alcohol, etc.

The various eye drop drugs with the therapeutical effect are purchased from the market.

Application of the above pharmaceutical preparation to preparation of drugs for preventing and treating asthenopia, eye dryness and distending pain, and eye angiosclerosis is also within the protection scope of the present disclosure.

Beneficial Effects: the present disclosure applies functions of activating blood circulation to dissipate blood stasis, eliminating swelling and relieving pain of the panax notoginseng, and is made into the dosage forms special for eye disease medication, and pharmacological experiments prove that the drug is safe and effective without stimulation, and has a curative effect on damages to eyes and vision caused by eye dryness, distending pain, asthenopia, eye angiosclerosis and the like, thereby having a better clinical application prospect.

### Detailed Description of the Invention

The present application is illustrated in detail below in combination with specific embodiments.

### Embodiment 1

### Preparation of Notoginsenoside Extract 1

20 kilograms of panax notoginseng are weighed, crushed into coarse powder through a wet method, and subjected to reflux extraction 3 times with 60% ethanol at weight ratios of 1:10, 1:8 and 1:8 respectively, 3 hours each time. An extracting solution is collected and filtered. A filtrate is taken to recycle the ethanol till no alcohol taste exists. Water is added to prepare a solution containing 0.5 to 1.0 g of a crude drug per 1 ml. The solution is loaded onto a macroporous adsorption resin column, and eluted with water and 70% to 90% ethanol liquid respectively. An 80% ethanol eluant is collected, concentrated and dried to obtain the notoginsenoside extract.

As determined with a total panax notoginseng saponins content determination method in Chinese Pharmacopoeia (I), the contents of all components are as follows: a content of a notoginsenoside R1 (C₄₇H₈₀O₁₈) is 6.2%, a content of a ginsenoside Rg1 (C₄₂H₇₂O₁₄) is 28.4%, a content of a ginsenoside Re (C₄₈H₈₂O₁₈) is 3.1%, a content of a ginsenoside Rb1 (C₅₄H₉₂O₂₃) is 32.7%, and a content of a ginsenoside Rd (C₄₈H₈₂O₁₉) is 5.8%.

### Embodiment 2

### Preparation of Notoginsenoside Extract 2

20 kilograms of radix notoginseng are weighed, crushed into coarse powder through a wet method, and subjected to reflux extraction 3 times with 70% ethanol at weight ratios of 1:10, 1:8 and 1:8 respectively, 3 hours each time. An extracting solution is collected and filtered. A filtrate is taken to recycle the ethanol till no alcohol taste exists. Water is added to prepare a solution containing 0.5 to 1.0 g of a crude drug per 1 ml. The solution is loaded onto a macroporous adsorption resin column, and eluted with water and 70% to 90% ethanol liquid respectively. An 80% ethanol eluant is collected, concentrated and dried to obtain the notoginsenoside extract.

As determined with a total panax notoginseng saponins content determination method in Chinese Pharmacopoeia (I), the contents of all components are as follows: a content of a notoginsenoside R1 (C₄₇H₈₀O₁₈) is 8.3%, a content of a ginsenoside Rg1 (C₄₂H₇₂O₁₄) is 31.4%, a content of a ginsenoside Re (C₄₈H₈₂O₁₈) is 7.1%, a content of a ginsenoside Rb1 (C₅₄H₉₂O₂₃) is 34.7%, and a content of a ginsenoside Rd (C₄₈H₈₂O₁₉) is 6.8%.

### Embodiment 3

### Preparation of Notoginsenoside Extract 3

20 kilograms of panax notoginseng are weighed, crushed into coarse powder through a wet method, and subjected to reflux extraction 3 times with 80% ethanol at weight ratios of 1:10, 1:8 and 1:8 respectively, 3 hours each time. An extracting solution is collected and filtered. A filtrate is taken to recycle the ethanol till no alcohol taste exists. Water is added to prepare a solution containing 0.5 to 1.0 g of a crude drug per 1 ml. The solution is loaded onto a macroporous adsorption resin column, and eluted with water and 70% to 90% ethanol liquid respectively. An 80% ethanol eluant is collected, concentrated and dried to obtain the notoginsenoside extract.

As determined with a total panax notoginseng saponins content determination in Chinese Pharmacopoeia (I), the contents of all components are as follows: a content of a notoginsenoside R1 (C₄₇H₈₀O₁₈) is 5.5%, a content of a ginsenoside Rg1 (C₄₂H₇₂O₁₄) is 31.9%, a content of a ginsenoside Re (C₄₈H₈₂O₁₈) is 4.2%, a content of a ginsenoside Rb1 (C₅₄H₉₂O₂₃) is 34.9%, and a content of a ginsenoside Rd (C₄₈H₈₂O₁₉) is 6.2%.

### Embodiment 4

### Notoginsenoside Eye Drop

100 g of the notoginsenoside extract prepared in Embodiment 1 is taken to be dissolved in 300 ml of normal saline. 0.3 g of activated carbon is added. A mixed solution is stirred, filtered after still standing for 8 hours, and finely filtered with a 0.22 µm film. A mixed solution is diluted to reach a constant volume of 1000 ml, sealed, sterilized at a high temperature and subpackaged into eye drop bottles, 1 ml per bottle, to obtain 1000 bottles of eye drops.

### Embodiment 5

### Notoginsenoside Eye Pad

The eye drop prepared in Embodiment 4 is taken to infiltrate 100 square centimeters of a non-woven fabric to obtain the notoginsenoside eye pad.

### Embodiment 6

### Notoginsenoside Eye Ointment

Medical yellow vaseline, glycerine and lanolin are taken and evenly mixed at a proportion of 8:2:1 to prepare an eye ointment matrix. 30 g of the notoginsenoside extract prepared in Embodiment 1 is added into 3000 g of the matrix, and evenly mixed to obtain the notoginsenoside eye ointment. The notoginsenoside eye ointment is subpackaged in a sterile state, 3 g per bottle, to obtain 1000 bottles.

### Embodiment 7

### Notoginsenoside Hydrogel

30 g of 2% carbomer gel, 5 g of glycerine, 0.5 g of hyaluronic acid and 45 g of purified water are taken and evenly mixed. Then 20 g of the notoginsenoside extract prepared in Embodiment 1 and 0.1 g of ethylparaben are added and evenly stirred by a homogenizer. Vacuum debubbling and bottling are conducted to obtain the notoginsenoside hydrogel.

### Embodiment 8

### Notoginsenoside Artificial Tears

20 g of a sterile solution containing 50 mg of a notoginsenoside per ml is added into 80 g of a sterile solution containing 15 mg of polyvinyl alcohol per ml and evenly mixed and filtered to obtain the notoginsenoside artificial tears.

### Embodiment 9

20.0 g of the notoginsenoside extract prepared in Embodiment 1 is weighed precisely. 980.0 g of purified water is added to prepare a solution with a concentration of 2% to obtain an eye drop. Then non-woven fabrics are infiltrated with the solution and then pasted to eye rims, which may relieve eye fatigue and sour and distending feelings of eyes.

### Embodiment 10

The eye drop prepared in Embodiment 9 is taken and mixed with artificial tears at 1:1, and is then dripped into eyes, which can eliminate discomfort caused by eye dryness and distending pain and eye angiosclerosis. The artificial tears are composed of one or more of anhydroglucose-70, polyethylene glycol 400, a sodium carboxymethyl cellulose eye drop, vitamin A, carbomer sorbic acid, sodium hyaluronate, polyvinyl alcohol, etc.

### Embodiment 11

The eye drop prepared in Embodiment 9 is taken and mixed with a commercially available eye drop at 1:1, and is then dripped into eyes, which can also eliminate discomfort caused by eye dryness and distending pain and eye angiosclerosis when treating various phlegm syndrome diseases of the eyes.

### Embodiment 12

### Asthenopia Relieving Drug Stimulation Experiment 1

The notoginsenoside extract prepared in Embodiment 1 is pipetted precisely. Purified water is added to prepare different concentration gradients of solutions to obtain experimental eye drops.

1. Grouping: the right eye of each rabbit is dosed with the experimental eye drops (according to the concentration gradients), and the left eye of each rabbit is dosed with equivalent normal saline.

Dosage Way: lower eyelids are pulled gently, and dosed with 30 µl of the experimental eye drops by a pipette, and then eyes are closed for five seconds.

Stimulation Dosage Frequency: nine times a day, and dosage once every 1 hour.

Dosage is conducted for one day totally. 1 hour after last dosage, observation is conducted. Observation results are recorded.

Grouping Standards: A total of 8 rabbits are grouped into a group A and a group B at random with 4 rabbits in each group, and four different concentrations are set in each group for experiments. Stimulation experiments and pharmacological experiments are conducted synchronously, and both are: right eyes are dosed with the experimental eye drops, and left eyes are dosed with the equivalent normal saline as control. Observed data are based on left eye control. For scoring standards, reference is made to a Draize scoring table. Observation results are shown in Table (1).

**Rabbit Stimulation Experiment Scoring Table (1)**

| | A1 right eye | A2 right eye | A3 right eye | A4 right eye | B1 right eye | B2 right eye | B3 right eye | B4 right eye |
|---|---|---|---|---|---|---|---|---|
| Conce ntratio ns | 10% | 5% | 2.5% | 1.25% | 10% | 5% | 2.5% | 1.25% |
| Situati ons | Upper eyelid edema; faint yellow colloidal secretions; and cornea bottom color deepening. | Upper eyelid edema; a small number of faint yellow colloidal secretions; and blood streaks at the corneas. | Slight eyelid edema; colloidal secretions; and blood streaks at the corneas and eyelids. | No eye edema; faint yellow secretions; and a small number of blood streaks at the corneas. | Slight upper eyelid edema; and faint yellow colloidal secretions. | Eyelid edema; and a small number of faint yellow colloidal secretions. | Slight eyelid edema; a small number of white secretions; and a small number of blood streaks at the corneas. | No eye edema; few faint yellow waxy secretions; and a small number of blood streaks at the corneas. |
| Scores | 4 | 3 | 3 | 2 | 3 | 2 | 3 | 2 |

| | | | | | | | | |
|---|---|---|---|---|---|---|---|---|
| Note: all records in the table are the left eye situations in comparison with right eye blank controls, and the scores of 3 or below are considered as no stimulation. | | | | | | | | |

2. After the rabbits are recovered completely, the concentration gradients are changed. The stimulation experiments are repeated once according to the above processes. Observation results are shown in Table (2).

**Rabbit Stimulation Experiment Scoring Table (2)**

| | A1 right eye | A2 right eye | A3 right eye | A4 right eye | B1 right eye | B2 right eye | B3 right eye | B4 right eye |
|---|---|---|---|---|---|---|---|---|
| Conc entrat ions | 2% | 1.5% | 0.8% | 1.25% | 2% | 1.5% | 0.8% | 1.25% |
| Situat ions | Being consistent with the left eye. | No eye edema; and a small number of blood streaks at the corneas. | No eye edema; colloidal secretions; and a small number of blood streaks at the corneas. | No eye edema; a small number of faint yellow secretions; and a small number of blood streaks at the corneas. | No eye edema; colloidal secretions; and a small number of blood streaks at the corneas. | Being consistent with the left eye. | No eye edema; and a small number of blood streaks at the corneas. | Being consistent with the left eye. |
| Score s | 0 | 1 | 2 | 2 | 2 | 0 | 1 | 0 |

### Asthenopia Relieving Drug Stimulation Experiment 2

Dosage Concentration: 1.25%.

Grouping: the right eye of each rabbit is dosed with the experimental eye drops, and the left eye of each rabbit is dosed with equivalent normal saline.

Dosage Way: lower eyelids are pulled gently, and dosed with 30 µl of the experimental eye drops by a pipette, and then eyes are closed for five seconds.

Stimulation Dosage Frequency: nine times a day, and dosage once every 1 hour.

Dosage is conducted for five days totally. 1 hour after last dosage every day, observation is conducted. Observation results are shown in Table (3).

**Rabbit Stimulation Experiment Scoring Table (3)**

| | A1 right eye | A2 right eye | A3 right eye | A4 right eye | B1 right eye | B2 right eye | B3 right eye | B4 right eye |
|---|---|---|---|---|---|---|---|---|
| First day | No eye edema; and a small number of blood streaks at the corneas. | Being consistent with the left eye. | No eye edema; and a small number of blood streaks at the corneas. | No eye edema; and a small number of faint yellow secretions. | No eye edema; and colloidal secretions. | A small number of colloidal secretions. | No eye edema; and a small number of blood streaks at the corneas. | Being consistent with the left eye. |

| Scores | 1 | 0 | 1 | 1 | 1 | 1 | 1 | 0 |
|---|---|---|---|---|---|---|---|---|
| Second day | No eye edema; and a small number of blood streaks at the corneas. | Being consistent with the left eye. | No eye edema; and a small number of blood streaks at the corneas. | No eye edema; and a small number of faint yellow secretions. | No eye edema; and colloidal secretions. | A small number of colloidal secretions. | No eye edema; and a small number of blood streaks at the corneas. | Being consistent with the left eye. |

| Scores | 1 | 0 | 1 | 1 | 1 | 1 | 1 | 0 |
|---|---|---|---|---|---|---|---|---|
| Third day | No eye edema; and a small number of blood streaks at the corneas. | Being consistent with the left eye. | No eye edema; and a small number of blood streaks at the corneas. | No eye edema; and a small number of faint yellow secretions. | No eye edema; and faint yellow colloidal secretions. | Being consistent with the left eye. | No eye edema; and a small number of blood streaks at the corneas. | Being consistent with the left eye. |

| Scores | 1 | 0 | 1 | 1 | 1 | 0 | 1 | 0 |
|---|---|---|---|---|---|---|---|---|
| Fourth day | No eye edema; and a small number of blood streaks at the corneas. | Slight eyelid edema. | No eye edema; and a small number of blood streaks at the corneas. | No eye edema; and a small number of faint yellow secretions. | No eye edema; and faint yellow colloidal secretions. | Being consistent with the left eye. | No eye edema; faint yellow secretions; and a small number of blood streaks at | Being consistent with the left eye. |
| | | | | | | | the corneas. | |

| Scores | 1 | 1 | 1 | 1 | 1 | 0 | 2 | 0 |
|---|---|---|---|---|---|---|---|---|
| Fifth day | No eye edema; and a small number of blood streaks at the corneas. | Being consistent with the left eye. | No eye edema; and a small number of blood streaks at the corneas. | No eye edema; a small number of faint yellow secretions; and a small number of blood streaks at the corneas. | No eye edema; and a small number of blood streaks at the corneas. | Being consistent with the left eye. | No eye edema; faint yellow secretions; and a small number of blood streaks at the corneas. | Being consistent with the left eye. |
| Scores | 1 | 0 | 1 | 2 | 1 | 0 | 2 | 0 |

Results of the above stimulation experiments show that the notoginsenoside eye drop drug is safe for rabbit eyes, and has no toxic and stimulation effects.

### Embodiment 13

### Asthenopia Relieving Pathological Experiment 1

### I. Materials and Methods:

(1) Experimental Animals and Grouping: 7 New Zealand rabbits are divided into 3 groups, 2 of the rabbits (numbers A and B) are in a normal group with five eyeballs in total, for the remaining 5 rabbits (numbers C, D, E, F and G), the left eyes are dosed with normal saline and used as a model group, and right eyes are dosed with a total panax notoginseng saponin eye drop and used as a dosage group.
(2) Experimental Materials: Rose Bengal, the total panax notoginseng saponin extract, and the normal saline.
(3) Submission Organs: rabbit eyeball HE slices (if submission samples are the left eyes, L is added behind their numbers, and if submission samples are the right eyes, R is added behind their numbers, for example, AL refers to the left eye of the rabbit A).
(4) Checking Method: the rabbits in the model group and the dosage group are injected with 5 ml of the 5% Rose Bengal at ear veins, a tropicamide eye drop is taken as a mydriatic eye drop, a procaine eye drop is taken as a local anesthetic, a green laser (532 nm) irradiates the eyes for modeling, and the normal group is not treated. The dosage group uses the 1.25% total panax notoginseng saponin eye drop (dissolved with the normal saline) for eye dropping for 1 week, once a day, 35 ul each time, and the model group is dosed with the normal saline in parallel. After the experiments are completed, the eyeballs are anatomised. After fixation, parts that we need are got. The parts are dehydrated, embedded with paraffin, and sliced up (4 µm thick). Slices are subjected to HE staining. Each part of retinas is observed under an optical microscope to check whether there are lesions or not and check types and degrees of the lesions. According to the degrees of the lesions, the lesions are sequentially semi-quantified to be slight (0.5 score), mild (1 score), moderate (2 scores), and serious (3 scores), and no negative lesion tissue (0 score).

### Asthenopia Relieving Pathological Experiment 2

### Experimental Animals and Grouping the Same as Experiment 1

(1) 4 normal eyeballs are clear in structures of all layers and have no obvious lesion. A retina under an optical microscope is composed of 10 layers of structures: sequentially from inside to outside, (1) an internal limiting membrane, which is extremely thin and has no cell; (2) a nerve fiber layer, which consists of non-medullated axons of ganglion cells and is narrow and lamellar; (3) a ganglion cell layer, which consists of cell bodies of the ganglion cells, wherein the cells are mutually connected and are single-layer cells; (4) an inner plexiform layer, which is composed of axons of bipolar cells and axons of the ganglion cells and has no obvious cell; (5) inner nuclear layers, which are composed of the bipolar cells connecting cone cells and rod cells and are about 4 layers; (6) an outer plexiform layer, which is formed by matching foot processes of retinal cells with dendrites of the bipolar cells mutually, and has no obvious cell; (7) outer nuclear layers, where cell nucleuses of visual cells are located, and which have about 8 to 10 layers of cells; and (8) to (10): sequentially an external limiting membrane, a visual cell layer and a retinal pigment epithelium (no pigment for rabbits).

### (2) Model Group (5 Rabbits):

Retinas are clear in structures of all layers. As for nerve fiber layers, nerve fiber layers of retinas of 3 rabbits have mild edema, with symptoms that tissues are loose, and vacuoles are formed in obvious parts. As for ganglion cell layers, the number of cells of a ganglion cell layer of one rabbits is reduced, in which there are a very few karyopyknosis cells. There are a very few karyopyknosis cells in ganglion cell layers of another 2 rabbits. As for inner nuclear layers, the number of cells of inner nuclear layers of retinas of 3 rabbits is reduced slightly, and 2 rabbits have mild edema.

### (3) Dosage Group (5 Rabbits)

Retinas are clear in structures of all layers, and have no obvious lesion in ganglion cell layers. As for nerve fiber layers, a nerve fiber layer of the retina of one rabbits has mild edema. As for ganglion cell layers, there are a few karyopyknosis cells in the ganglion cell layers of 3 rabbits. There is no obvious lesion in inner nuclear layers, outer nuclear layers and other layers.

### Asthenopia Relieving Pathological Experiment 3

### Experimental Animals and Grouping the Same as Experiment 1

1. In the experiment, 5% Rose Bengal is utilized to be injected into ear veins, and a laser irradiates eyes for modeling. Lesions of retinas of rabbits in a model group are mainly manifested as nerve fiber layers or inner nuclear layers edema, karyopyknosis cells, and reduced number of cells of the inner nuclear layers.
2. After drug application, the number of cases of the above lesions is significantly reduced, and the degrees are significantly reduced. It shows that the drug is safe and effective.

**Retinal Lesion Pathological Check Table**

| Groups | Normal group | | | | Model group | | | | | Dosage group | | | | |
|---|---|---|---|---|---|---|---|---|---|---|---|---|---|---|
| Parts | AL | AR | BL | BR | CL | DL | EL | FL | GL | CR | DR | ER | FR | GR |
| Internal limiting membrane | | | | | | | | | | | | | | |
| Nerve fiber layer edema | | | | | 1 | 1 | | | 1 | | | | 1 | |
| Ganglion cell reducing | | | | | | | | | 1 | | | | | |
| Karyopyknosis cell | | | | | | | 1 | 1 | 1 | | 1 | 1 | Seve ral | |
| Inner plexiform layer | | | | | | | | | | | | | | |
| Inner nuclear layer edema | | | | | | 1 | | 1 | | | | | | |
| Cell reducing | | | | | 1 | 1 | | | 1 | | | | | |
| Outer plexiform layer | | | | | | | | | | | | | | |
| Outer nuclear layer cell reducing | | | | | | | | | | | | | | |
| External limiting membrane | | | | | | | | | | | | | | |
| Visual cell layer | | | | | | | | | | | | | | |
| Total scores | 0 | | | | 2 | 3 | 1 | 2 | 4 | 0 | 1 | 0.5 | 1 | 0 |
| Mean values | | | | | 2.4 | | | | | 0.5 | | | | |
| SD | | | | | 1.1402 | | | | | 0 | | | | |
| Response rate | 100% | | | | | | | | | | | | | |
| Cure rate | 79% | | | | | | | | | | | | | |
| Significance | | | | | P<0.01 | | | | | | | | | |

The above experiments show that the total panax notoginseng saponin eye drop is safe for the rabbit eyes, and has a curative effect on damages to the eyes and vision caused by eye dryness, distending pain, asthenopia, eye angiosclerosis and the like.

## Claims

1. An ophthalmic pharmaceutical preparation, wherein an effective component of the ophthalmic pharmaceutical preparation is a notoginsenoside extract.

2. The ophthalmic pharmaceutical preparation according to claim 1, wherein a weight percentage content of the notoginsenoside extract in the pharmaceutical preparation is 0.1 to 30%.

3. The ophthalmic pharmaceutical preparation according to claim 2, wherein the weight percentage content of the notoginsenoside extract in the pharmaceutical preparation is 0.2 to 10%.

4. The ophthalmic pharmaceutical preparation according to claim 1, wherein the notoginsenoside extract is the only effective component, and the pharmaceutical preparation is prepared from 0.1 to 30% by weight of the notoginsenoside extract and 70 to 99.9% by weight of a pharmaceutically acceptable carrier and/or excipient.

5. The ophthalmic pharmaceutical preparation according to any one of claims 1 to 4, wherein the notoginsenoside extract contains the following components with a total content of 45 to 95%: a notoginsenoside R1, a notoginsenoside R2, aginsenoside Rg1, a ginsenoside Rg2, a ginsenoside Re, a ginsenoside Rb1, a ginsenoside Rb2, a ginsenoside Rd, a ginsenoside Rf, a ginsenoside Rc, a ginsenoside Rh1 and a gypenoside IX.

6. The ophthalmic pharmaceutical preparation according to claim 5, wherein the notoginsenoside extract contains the following components with a total content of 75 to 90%: the notoginsenoside R1, the notoginsenoside R2, the ginsenoside Rg1, the ginsenoside Rg2, the ginsenoside Re, the ginsenoside Rb1, the ginsenoside Rb2, the ginsenoside Rd, the ginsenoside Rf, the ginsenoside Rc, the ginsenoside Rh1 and the gypenoside IX.

7. The ophthalmic pharmaceutical preparation according to any one of claims 1 to 4, wherein in the notoginsenoside extract, a content of a notoginsenoside R1 is equal to or greater than 3.0%, a content of a ginsenoside Rg1 is equal to or greater than 15.0%, a content of a ginsenoside Re is equal to or greater than 1.0%, a content of a ginsenoside Rb1 is equal to or greater than 20.0%, and a content of a ginsenoside Rd is equal to or greater than 2.5%.

8. The ophthalmic pharmaceutical preparation according to claim 7, wherein in the notoginsenoside extract, the content of the notoginsenoside R1 is equal to or greater than 5.0%, the content of the ginsenoside Rg1 is equal to or greater than 25.0%, the content of the ginsenoside Re is equal to or greater than 2.5%, the content of the ginsenoside Rb1 is equal to or greater than 30.0%, and the content of the ginsenoside Rd is equal to or greater than 5%.

9. The pharmaceutical preparation according to claim 1, wherein the effective component, the notoginsenoside extract, is added into artificial tears or various eye drop drugs with a therapeutical effect.

10. Application of the pharmaceutical preparation according to claim 1 to preparation of drugs for preventing and treating asthenopia, eye dryness and distending pain, and eye angiosclerosis.
